# EUROPEAN PATENT SPECIFICATION

(11) **EP 1 037 928 B1**
(45) Date of publication and mention of the grant of the patent: **13.03.2002**
(21) Application number: 97952569.8
(22) Date of filing: 19.12.1997
(51) Int. Cl.: C08B 37/00, C08B 11/145, C08F 8/00, C08G 63/91, C08G 65/48, C08G 77/38, C08G 85/00

(54) **MULTI-CATIONIC POLYMERS**
MULTIKATIONISCHE POLYMERE
POLYMERES MULTICATIONIQUES

(43) Date of publication of application: 27.09.2000
(73) Proprietor: THE PROCTER & GAMBLE COMPANY, Cincinnati, Ohio 45202 (US)
(72) Inventor: BRYANT, Lonnie Liddell Jr., Momoyama-cho, Fushimi-ku, Kyoto 612 (JP); ONO, Masami, Higashinada-ku, Kobe 658 (JP)
(74) Representative: McKinney, Victoria
(86) International application number: US9723621
(87) International publication number: WO9932521

(56) References cited:
- EP-A- 0 212 145
- EP-A- 0 310 787
- WO-A-94/06406
- KURITA K. ET AL.: "Chitin/synthetic polymer hybrid materials: Efficient graft copolymerizations onto chitin derivatives" ADV. CHITIN SCI., vol. 1, 1996, pages 285-290, XP002076991
- H. CHERADAME ET AL.: "The cationic grafting of cellulose with 2-methyl-2-oxazoline" MAKROMOL. CHEM. MACROMOL. SYMP., vol. 6, 1986, pages 261-270, XP002076992
- I. IKEDA ET AL.: "Cationic graft polymerization of 2-oxazolines on cellulose derivatives" POLYMER JOURNAL, vol. 20, no. 3, 1988, pages 243-250, XP002076993
- I. IKEDA ET AL.: "Cationic graft polymerization of 2-oxazolines on poly(vinyl alcohol) derivatives" POLYMER-PLASTICS TECHNOLOGY AND ENGINEERING, vol. 28, no. 7-8, 1989, pages 877-890, XP002076994 usa

## Description

### TECHNICAL FIELD

The present invention relates to a multi-cationic polymer having a block cationic polyelectrolyte site. The present invention further relates to suitable methods of making the multi-cationic polymer.

### BACKGROUND

High molecular weight materials are used in various fields of industry. Some general uses of these materials include use as structural materials or composite materials, for coating, covering, barrier, and delivery.

However, many high molecular weight material generally have poor adhesion to surfaces such as fabrics, hair and skin, glass, metals, and filler materials. It is known in the art that such surfaces are generally slightly negatively charged. Material having positive charges are expected to have better deposition on such surfaces.

Polymers having multi-cationic sites are known in the art, such as in Saegusa et al. "Catalysis of Polymers Containing Quaternized Linear Poly(N-alkylethyleneimine) Chain in Aqueous-Organic Hetero-Phase Reactions" Polymer Journal, Vol. 11, No. 1, pp1-6, issued 1979.

The document WO-A-9406406 discloses cosmetic compositions for the skin or hair, nails, eyebrows and eyelashes comprising a dispersion of solid organic particles in a binder, the surface of the particles is coated with a multi cationic polymer, which contains primary, secondary, tertiary and/or quaternary ammonium groups, either as part of the polymer chain or on a lateral substituent.

The particles are easily dispersed, even in a fatty binder. The compositions have good stability and adhere well to the skin.

Based on the foregoing, there is a need for a polymer which has good adhesion to various surfaces, and thus suitable for use in products required to have good deposition to surfaces.

None of the existing art provides all of the advantages and benefits of the present invention.

### SUMMARY

The present invention is directed to a multi-cationic polymer comprising:
a) formulae (la): 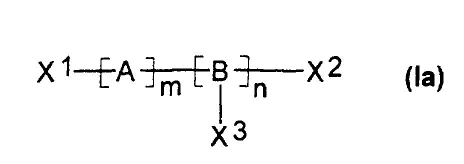 wherein [A]ₘ and [B]ₙ, individually, is a non-reacting polymeric substituent
   wherein m+n is an integer of 1 to about 100,000, where one of m or n can be 0; and at least one of X¹, X², and X³ is formulae (Ib): 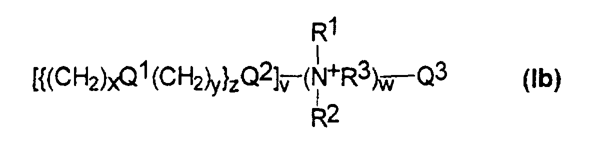 wherein Q¹ is oxygen, nitrogen, sulfur or methylene; x is an integer of 0 to about 20; y is an integer of 0 to about 20; z is an integer of 0 to about 20; Q² is CH₂ or any of the following formulae: 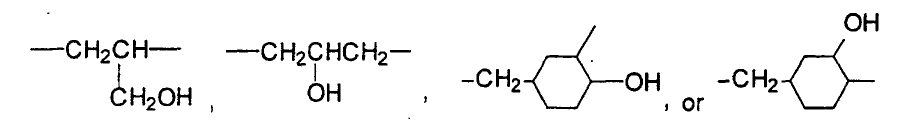 v is 0 or 1; R¹ is hydrogen, alkyl of 1 to 4 carbons, fluorocarbon of 1 to 4 carbons, phenyl, or benzyl; R² is alkyl of 1 to about 30 carbons, phenyl, benzyl, or phenethyl; R³ is ethylene or propylene; Q³ is OH, OR⁸, NH₂, NHR⁹, SH, SR¹⁰, COOH, COOR¹¹, or a halogen, wherein R⁸, R⁹, R¹⁰, and R¹¹ are alkyl or alkylene of 1 to about 20 carbons; and w is an integer of 1 to about 100; the remainder of X¹, X², and X³ being a non-reacting end group; and
b) a stoichiometric amount of an anion selected from the group consisting of anionic forms of sulfate, hydrosulfate, methylsulfate, carbonate, bicarbonate, chloride, bromide, iodide, and mixtures thereof.

The present invention is further directed to a suitable method of making the multi-cationic polymer.

These and other features, aspects, and advantages of the present invention will become evident to those skilled in the art from reading of the present disclosure.

### DETAILED DESCRIPTION

While the specification concludes with claims particularly pointing and distinctly claiming the invention, it is believed the present invention will be better understood from the following description.

All percentages herein are by weight of the compositions unless otherwise indicated.

All ratios are weight ratios unless otherwise indicated.

All percentages, ratios, and levels of ingredients referred to herein are based on the actual amount of the ingredient by weight, and do not include solvents, fillers, or other materials with which the ingredient may be combined as commercially available products, unless otherwise indicated.

As used herein, "comprising" means that other steps and other ingredients which do not affect the end result can be added. This term encompasses the terms " consisting of" and "consisting essentially of'.

All cited references are incorporated herein by reference in their entireties. Citation of any reference is not an admission regarding any determination as to its availability as prior art to the claimed invention.

### MULTI-CATIONIC POLYMER

The multi-cationic polymer of the present invention comprises:
a) formulae (la): 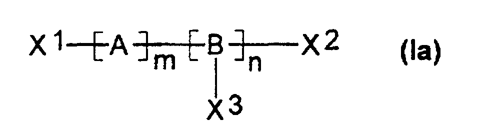 wherein [A]ₘ and [B]ₙ, individually, is a non-reacting polymeric substituent wherein m+n is an integer of 1 to about 100,000, where one of m or n can be 0; and at least one of X¹, X², and X³ is formulae (Ib): 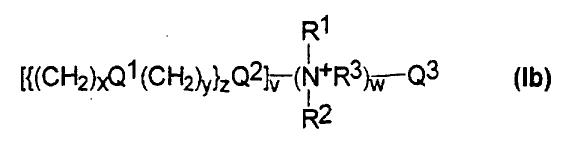 wherein Q¹ is oxygen, nitrogen, sulfur or methylene; x is an integer of 0 to about 20; y is an integer of 0 to about 20; z is an integer of 0 to about 20; Q² is CH₂ or any of the following formulae: 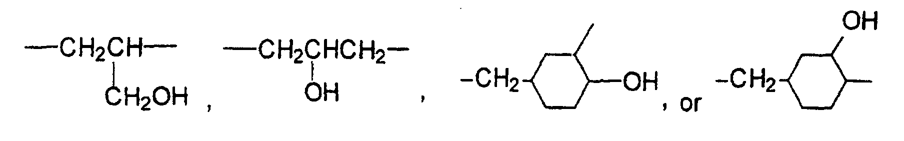 v is 0 or 1; R¹ is hydrogen, alkyl of 1 to 4 carbons, fluorocarbon of 1 to 4 carbons, phenyl, or benzyl; R² is alkyl of 1 to about 30 carbons, phenyl, benzyl, or phenethyl; R³ is ethylene or propylene; Q³ is OH, OR⁸, NH₂, NHR⁹, SH, SR¹⁰, COOH, COOR¹¹, or a halogen, wherein R⁸, R⁹, R¹⁰, and R¹¹ are alkyl or alkylene of 1 to about 20 carbons; and w is an integer of 1 to about 100; the remainder of X¹, X², and X³ being a non-reacting end group; and
b) a stoichiometric amount of an anion selected from the group consisting of anionic forms of sulfate, hydrosulfate, methylsulfate, carbonate, bicarbonate, chloride, bromide, iodide, and mixtures thereof.

The multi-cationic polymers herein comprise at least one of a block cationic polyelectrolyte site as represented by formulae (Ib) above. With such a structure, the multi-cationic polymers herein possess the characteristics of the original starting material polymer as well as characteristics of a cationic polymer. The structural design and molecular weight of the multi-cationic polymer herein may be selected by the artisan to obtain those according to the desired product to which the polymer is formulated. The multi-cationic polymer herein may terminate with an additional moiety or include adequate spacers as selected by the artisan to facilitate synthesis. The portions of the multi-cationic polymer herein which are not terminated with formulae (Ib) are terminated or end-capped with a non-reacting group such as alkyl or trimethylsilicon.

Units [A]ₘ and [B]_{n,} individually, are non-reacting polymeric substituents wherein m+n is an integer of 1 to about 100,000, where one of m or n can be 0. Unit [A] represents a polymeric substituent which has only one reacting site at the terminal of the polymer. Unit [B] represents a polymeric substituent which has a pendant reacting site at each unit. Units [A]ₘ and [B]_{n,} individually, may be copolymers; i.e.; they may be made of more than one repeating unit. Units [A] and [B], may also be included in random order. Suitable polymeric substituents for units [A]ₘ and [B]ₙ include polysiloxanes, polyalkylene oxides. polysaccharides, oligo(oxymethylene) dihydrates, oligo(esters) of hydroxy acids, diol oligo(urethanes), polymeric phenol alcohols, poly(furfuryl) derivatives, polyacrylates, polyesters, polyolefins, poly(vinyl alcohol)s, long alkyl fatty alcohols, polyols, and mixtures thereof. Exemplary polysilxoxanes include hydroxyalkyl-siloxanes, hydroxyalkoxyalkyt-siloxanes, and polyalkoxyl-siloxanes. Exemplary polyalkylene oxides include poly(methylene oxide), poly(ethylene oxide), poly(propylene oxide), poly(butylene oxide), monoalkylethers of poly(alkylene oxides). Exemplary polysaccharides include poly(β-xylopyranose), poly(maltose), cyclodextrins, celluloses, poly(isomaltose), poly(gentiobiose), poly(galactose), poly(mannose) and chitin. Exemplary oligo(oxymethylene) dihydrates include monoalkylethers of oligo(oxymethylene)dihydrates. Exemplary oligo(esters) of hydroxy acids include methyl, ethyl, propyl esters of poly(α-hydroxy acid), poly(β-hydroxy butyric acid), poly(β-hydroxy pivalic acid), poly(β-hydroxy butyric acid) isobutyl ester. Exemplary diol oligo(urethanes) include polyurethanes of diisocyanates, 1,4-butanediol, hexamethylene diisocyanide, and diglycols. Exemplary polymeric phenol alcohols include poly(phenol alcohols), poly(phenol dialcohols), poly(p-cresol monoalcohols), poly(p-tert-butylphenol alcohols), poly(o-hydroxydibenzyl ethers), and phenolformaldehyde resins. Exemplary poly(furfuryl) derivatives include poly(furfuryl) alcohols. Exemplary polyacrylates include poly(acrylonitrile) transformed by ethanolamine. Exemplary polyesters include polyneopentyl glycol adipate, polyethylene glycol isothalate, polyethylene glycol tetrachlorophthalate, polyethylene glycol sebacate, polybutane diol succinate, polydiethylene glycol succinate and poly diethylene glycol terepthalate. Exemplary polyolefins include poly(ethylene-co-vinyl acetate-co-allyl alcohol) and poly(ethylene-co-vinyl alcohol). Exemplary fatty alcohols and polyols include decanol, dodecanol, hexadecanol, and octadecanol. Preferably, units [A]m and [B]n are selected from the group consisting of polysiloxanes, polyalkylene oxides, polysaccharides, and mixtures thereof.

The characteristic block cationic polyelectrolyte site of the multi-cationic polymer is included in formulae (Ib): 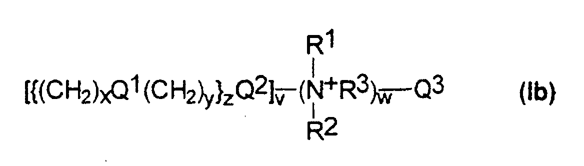 wherein at least one of X¹, X², and X³ is this end group, and the remainder are non-reacting end groups. The multi-cationic polymers herein may or may not have a plurality of block cationic polyelectrolyte sites. Q¹ is oxygen, nitrogen, sulfur or methylene; x is an integer of 0 to about 20; y is an integer of 0 to about 20; z is an integer of 0 to about 20; Q² is CH₂ or any of the following formulae: 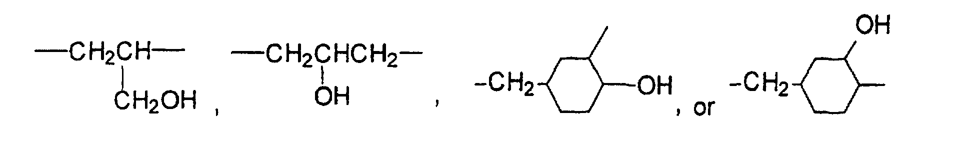 v is 0 or 1; R¹ is hydrogen, alkyl of 1 to 4 carbons, fluorocarbon of 1 to 4 carbons, phenyl, or benzyl; R² is alkyl of 1 to about 30 carbons, phenyl, benzyl, or phenethyl; R³ is ethylene or propylene; and w is an integer of 1 to about 100; Q³ is OH, OR⁸, NH₂, NHR⁹, SH, SR¹⁰, COOH, COOR¹¹, or a halogen, wherein R⁸, R⁹, R¹⁰, and R¹¹ are alkyl or alkylene of 1 to about 20 carbons.

Formulae (Ib) comprises the block cationic polyelectrolyte site represented by the following portion of formulae (Ib): 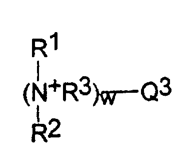 Preferably, R¹ is hydrogen, methyl, ethyl, phenyl, or benzyl, more preferably methyl or ethyl; R² is alkyl of 1 to about 30 carbons, more preferably methyl or ethyl; R³ is ethylene or propylene; w is 3 to about 100; and Q³ is OH.

The block cationic polyelectrolyte site represented by formulae (Ib) may or may not include a spacer {(CH₂)ₓQ¹(CH₂)_{y}}_{z} for facilitating synthesis. When the starting material is a polysiloxane, the spacer is preferably included. When the spacer is included, Q¹ is preferably oxygen or methylene, x is preferably 0 to about 5; y is preferably 0 to about 5; and z is an integer of preferably 0 to about 10.

The multi-cationic polymers herein have a stoichiometric amount of an anion selected from the group consisting of anion forms of sulfate, hydrosulfate, methylsulfate, carbonate, bicarbonate, chloride, bromide, iodide, and mixtures thereof

### RING-OPENING POLYMERIZATION

The multi-cationic polymers of the present invention can be prepared by a synthesis route comprising three stages.

The first stage comprises ring-opening polymerization of an activated starting polymer.

The first stage may further comprise a preliminary stage of preparing an activated starting polymer. The activated starting polymer is a polymer compound having its terminals substituted with a sulfonic ester or a halogen, preferably tosyl or mesyl sulfonic ester groups.

The starting material to make this activated starting polymer can be any linear or branched starting material having hydroxy functionality, wherein any other existing functionality are non-reactive with the below mentioned activating agents, nor the below mentioned cyclic iminoethers. The starting material has at least one of the following as an end group, the other portions being non-reactive: 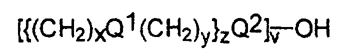 wherein Q¹ is oxygen, nitrogen, sulfur or methylene; x is an integer of 0 to about 20; y is an integer of 0 to about 20; z is an integer of 0 to about 20. The starting material can be any polymer known in the art so long as at least one of the terminals is hydroxy. Suitable starting material are those listed under polymeric substituents for units [A]ₘ and [B]ₙ above.

When the starting material is a polysiloxane, the starting material is preferably a hydroxyalkyl-siloxane, hydroxyalkoxyalkyl-siloxane, and polyalkoxylsiloxane. Examples of commercially available starting silicone materials are; X-22-160AS, KF-6001, KF-6002, KF-6003, X-22-4015, X-22-4901, KF-353, KF-354, KF-355, KF-945, and KF-6011 supplied by Shin-Etsu Chemical, SF8427, BY16-005, BY16-007, SH3746, SF8428, SH3771, BY16-036, BY16-027, and BY16-848 supplied by Toray Dow Corning Silicone, TSF4750, TSF4751, XF42-B0970, SF1188, SF1288, and SF1388 supplied by Toshiba Silicone.

When the starting material is a polyalkylene oxide, the starting material is preferably a polyethylene oxide, polypropylene oxide, polybutylene oxide, and monoalkyethers of the above. Examples of commercially available starting polyalkylene oxide materials are; 18,198-6, 18,199-4, 18,200-1, 37,277-3. 37,280-3 as specified in "Catalog Handbook of Fine Chemicals 1996-1997" available from Aldrich Chemical Company, and 167-09045, 164-09055, 161-09065, 168-09075, 165-09085, 162-09095, 165-09105, 162-09115, 169-09125, 163-17575, 167-05885, 164-05895, 167-17615, 164-17625, and 167-05905 as specified in "Wako Catalog 1996" available from Wako Pure Chemicals Industry.

When the starting material is a polysaccharide, the starting material is preferably a poly(β-xylopyranose), poly(maltose), cyclodextrins, celluloses, poly(isomaltose), poly(gentiobiose), poly(galactose), poly(mannose) and chitin. Examples of commercially available starting polysaccharide materials are; 556-32961, 554-37381, 047-23611, 536-02831, 033-15221, 033-08332, 030-08342, 034-08345, 031-10641 as specified in "Wako Catalog 1996" available from Wako Pure Chemicals Industry, 31,069-7, 18,095-5, 32,807-3, 43,522-8, 18,465-9, 43,524-4, 31,069-7, 41,795-5, 44,869-9, and 41,796-3 as specified in "Catalog Handbook of Fine Chemicals 1996-1997" available from Aldrich Chemical Company.

An embodiment of preparation of an activated starting polymer is shown in the following scheme: 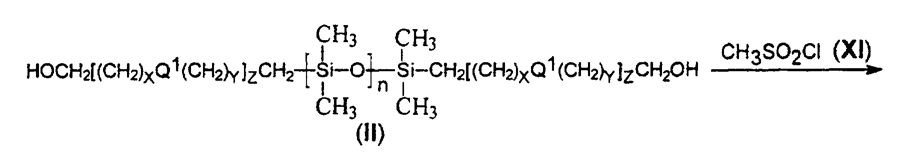 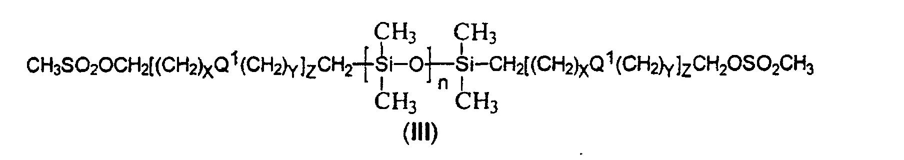 The starting material as exemplified by dihydroxy-terminated polydimethylsiloxane as shown by formula (II) is subjected to the reaction with an activating agent to obtain an activated starting polymer as shown by formula (III). The activating agent is selected from the group consisting of sulfonic halide, wherein halide is a chloride, bromide, or iodide, P(Hal)₃, P(Hal)₅ and SO₂(Hal)₂ or mixtures thereof; wherein (Hal) is a halogen selected from chloride, bromide, or iodide. Preferable activating agents are tosyl chloride and mesyl chloride. Mesyl chloride is exemplified as formula (XI).

Suitable mediums for carrying out this first stage of synthesis include inert solvents which provide an anhydrous condition. Exemplary inert solvents are absolute (hereinafter referred to as "abs.") tetrahydrofuran (hereinafter referred to as "THF"), abs. benzene, abs. toluene, abs. triethylamine, abs. pyridine, and mixtures thereof. Preferably, the medium contains a Lewis base. Exemplary Lewis bases include those which are liquid and solid such as abs. triethylamine, abs. pyridine, dimethylamino pyridine (hereinafter referred to as "DMAP"), and 1,8-diazabicyclo[5.4.0.]-7-undecene (hereinafter referred to as "DBU"). Abs. triethylamine and abs. pyridine act both as a solvent and a liquid Lewis base, and thus suitable. Abs. triethylamine is a highly suitable liquid Lewis base. More preferably, the medium contains at least a stoichiometric amount to the activating agent of a Lewis base, as it provides good yield. Still preferably, the medium contains: a volatile solvent such as abs. THF, abs. benzene, abs. toluene and mixtures thereof; and a stoichiometric amount to the activating agent of a liquid Lewis base such as abs. triethylamine, abs. pyridine, and mixtures thereof. The reaction can be carried out at room temperature at atmospheric pressure.

Alternatively, the activated starting polymer can be any linear or branched polymer having halogen or epoxy functionalities, wherein any other existing functionalities are non-reactive with the below mentioned cyclic iminoethers. The starting material has at least one of the following as an end group, the other portions being non-reactive:

{(CH₂)ₓQ¹(CH₂)_{y}}_{z}―X⁴

wherein Q¹ is oxygen, nitrogen, sulfur or methylene; x is an integer of 0 to about 20; y is an integer of 0 to about 20; z is an integer of 0 to about 20; and X⁴ is a halide or an epoxy of the following formulae: 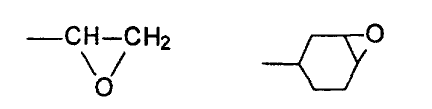 The activated starting polymer can be any known in the art so long as it has at least one halogen or epoxy functionality. The epoxy functionality can be a glycidyl-type or oxirane-type. Preferably, the activated starting polymer has halogen functionalities. When these halogen terminated activated starting polymers are used directly for ring-opening polymerization, the above mentioned reaction with the activating agents are unnecessary.

Examples of commercially available polysiloxane starting polymers with halogen functionalities which may be used directly as activated starting polymer are; LMS-152 supplied by AZmax Co., Ltd., TSL9236, TSL9276, TSL9226, and TSL9206 supplied by Toshiba Silicone. Commercially available siloxane compounds having epoxy functionalities are; KF-105, X-22-163A, X-22-163B, X-22-163C, KF-1001, KF-101, X-22-169AS, X-22-169B, KF-102, and X-22-173DX supplied by Shin-Etsu Chemical, SF8411, SF8413, BY16-855, BY16-855B, BY16-839, SF8421EG, and BY16-845 supplied by Toray Dow Corning Silicone, YF3965, XF42-A4439, TSF4730, XF42-A4438, XC96-A4462, XC96-A4463, XC96-A4464, XF42-A5041, TSL9946, TSL9986, and TSL9906 supplied by Toshiba Silicone.

Examples of commercially available polyalkylene oxide starting polymers with halogen functionalities which may be used directly as an activated starting polymer is; 18,186-2 as specified in "Catalog Handbook of Fine Chemicals 1996-1997" available from Aldrich Chemical Company.

The activated starting polymer either obtained by the preliminary reaction or obtained commercially is polymerized through a reaction known as ring-opening polymerization, an embodiment of which is shown in the following scheme: 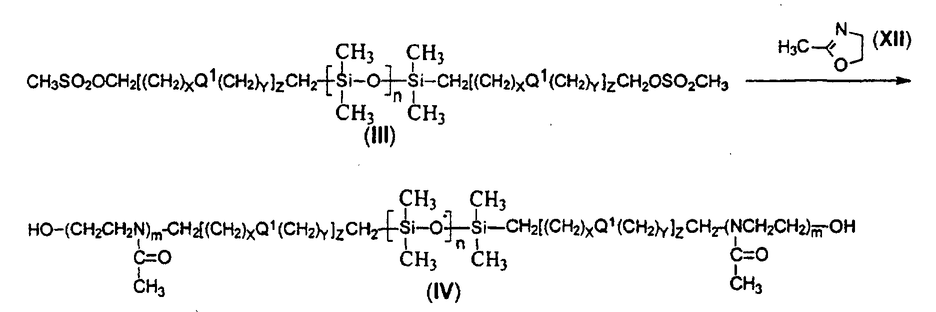 This can be done by subjecting the activated starting polymer as exemplified by formula (III) with a cyclic iminoether to obtain a polyamide intermediate exemplified by formula (IV). The cyclic iminoether is selected from the group consisting of 2-substituted-2-oxazoline, 2-substituted-5,6-dihydro-4H1,3-oxazine, and mixtures thereof. Preferable cyclic iminoethers are 2-Z'-2-oxazoline and 2-Z'-oxazine wherein Z' is a hydrogen, alkyl, or fluorocarbon having 1 to 10 carbons, phenyl and benzyl, still preferable are 2-alkyl-2-oxazoline wherein the alkyl is made of 1 to 3 carbons, as exemplified as formula (XII). The tosyl, mesyl or halide group of the activated starting polymer (III) is known to be a leaving group on the attack by various nucleophilic monomers. The tosyl and mesyl groups are preferable leaving groups. Thus, the activated starting polymer (III) is utilized as a macromolecular initiator for the synthesis of a block copolymer through nucleophilic reaction toward the tosylate or mesylate ester function or halide at the functionality by the cyclic iminoether which acts as a nucleophilic monomer. The polyamide intermediate (IV) thus obtained has concentrated polyamide moieties at functionalities of the polymer which had originally been activated.

Suitable mediums for carrying out this first stage of synthesis include inert solvents which provide an anhydrous condition. Exemplary inert solvents are abs. acetonitrile, abs. THF, abs. benzene, abs. toluene, abs. triethylamine, abs. pyridine, and mixtures thereof. Preferably, the medium contains a Lewis base. Exemplary Lewis bases include those which are liquid and solid such as abs. triethylamine, abs. pyridine, dimethylamino pyridine (hereinafter referred to as "DMAP"), and 1,8-diazabicyclo[5.4.0.]-7-undecene (hereinafter referred to as "DBU"). Abs. triethylamine and abs. pyridine act both as a solvent and a liquid Lewis base, and thus suitable. Abs. triethylamine is a highly suitable liquid Lewis base. More preferably, the medium contains at least a stoichiometric amount to the activated starting polymer as exemplified by activated siloxane (III) of a Lewis base. It has been found that the existence of this amount of Lewis base significantly raises the conversion yield of the activated starting polymer to the resulting polyamide intermediate. Thus, the number of amide moieties of the present polymers (number "m" in the structure) can be controlled with significantly improved precision to provide the targeted polymer at high yield. Still preferably, the medium contains: a volatile solvent such as abs. acetonitrile, abs. THF, abs. benzene, abs. toluene and mixtures thereof; and a stoichiometric amount to the activating agent of a liquid Lewis base such as abs. triethylamine, abs. pyridine, and mixtures thereof. A highly preferred medium is an acetonitrile solution of at least a stoichiometric amount to the activated starting polymer of abs. triethylamine. The reaction can be carried out at room temperature or elevated temperature at around atmospheric pressure.

After the ring-opening polymerization is completed, the obtained reactant is treated with an alkali or alkali salt to obtain the polyamide intermediate (IV). Such alkali or alkali salt include sodium carbonate, sodium hydrogen carbonate, potassium carbonate, potassium hydrogen carbonate, amines in an inert solvent, and metal or tetra-alkyl ammonium salts of organic acids. When the reactant is treated with aqueous alkaline such as sodium carbonate, the finally obtained polymer will have a hydroxy terminal. When the reactant is treated with amines in an inert solvent, the finally obtained polymer will have amine or ammonium terminated terminals. When the reactant is treated with salts of organic acids, the finally obtained intermediate will have carboxylate or ester terminals.

### REDUCTION OR HYDROLYSIS

The second stage comprises reduction or hydrolysis of the obtained polyamide intermediate (IV).

Reduction can be done, for example, by reacting the polyamide intermediate with a reducing agent such as LiAl(OR)ₓH₄₋ₓ wherein R is methyl, ethyl, or isopropyl, and x is 0 or an integer from 1 to 3; preferably LiAlH₄ (lithium aluminum hydride) to obtain a reduced polyamide intermediate as exemplified by formula (V) as shown in the following scheme: 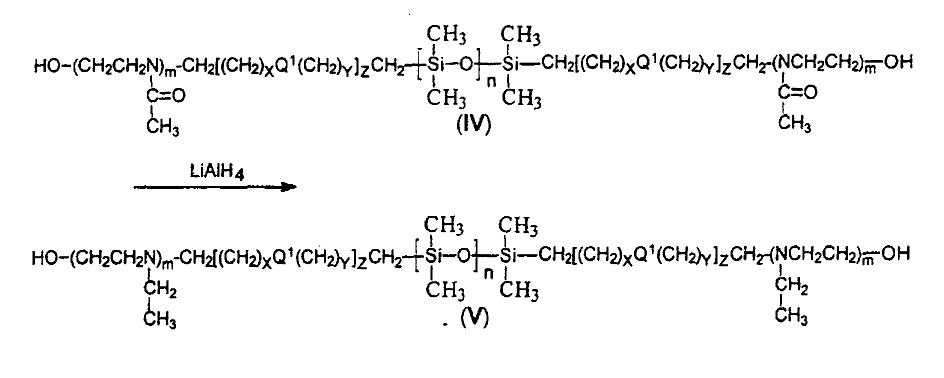 Suitable mediems for carrying out such reduction include inert solvents such as abs. THF and abs. diethylether. Reduction can be alternatively done by reacting the polyamide polymer with an agent such as (C₂H₅)₃O⁺BF₄⁻, Cl₃SiH, Cl₃SiOH, Cl₃SiOCH₃, Cl₃SiOC₂H₅, (CH₃)₃SiCl, or dimethylsulfate in an inert solvent such as n-hexane or CH₂Cl₂, followed by treatment with NaBCNH₃or NaBH₄ in diethylether, ethanol, or THF.

Hydrolysis can be done, for example, by reacting the polyamide intermediate with aqueous and alcohol/aqueous solutions of strong acids and alkalis such as HCI, NaOH, KOH, or NaHCO₃ to obtain a hydrolyzed polyamide intermediate as exemplified by formula (VI) as shown in the following scheme: 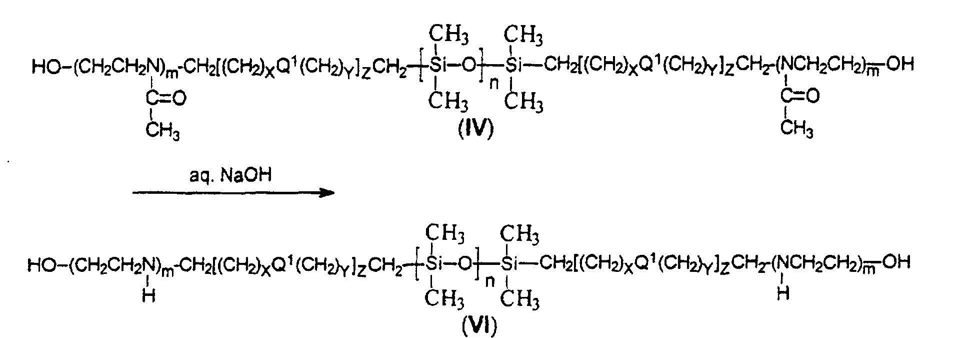 Hydrolysis can be alternatively done by reacting the polyamide intermediate with an agent such as (C₂H₅)₃O⁺BF₄⁻, Cl₃SiH, Cl₃SiOH, Cl₃SiOCH₃, Cl₃SiOC₂H₅, (CH₃)₃SiCl, H₂N-NH₂, or dimethylsulfate in an inert solvent such as n-hexane abs. THF, diethylether or CH₂Cl₂, followed by treatment with diluted aqueous solutions of acids and alkalis such as HCI, NaOH, KOH, NaHCO₃, K₂CO₃, Na₂CO₃, or KHCO₃.

### QUATERNIZATION

The third stage comprises quaternization of the obtained reduced polyamide intermediate (V) or hydrolyzed polyamide intermediate (VI) to obtain a multi-cationic polymer of the present invention. This can be carried out by adding a quaternizing agent. Quaternizing agents are selected from the group consisting of monomethylsulfuric acid, dimethylsulfate, diethylsulfate, dimethylcarbonate, methylchloride, methyliodide, methylbromide, ethylchloride, ethyliodide, ethylbromide, benzylchloride, benzylbromide, benzyliodide, sulfuric acid, carboxylic acid, hydrochloric acid, hydrobromic acid, hydroiodic acid, and mixtures thereof. It is known in the art that, when quaternization is conducted with quaternizing agents which provide positively charged alkyls such as monomethylsulfuric acid, dimethylsulfate, diethylsulfate, dimethylcarbonate, methylchloride, methyliodide, methylbromide, ethylchloride, ethyliodide, ethylbromide, benzylchloride, benzylbromide, and benzyliodide, the quaternized cite is stable and pH independent. Quaternization which provides positively charged alkyls is exemplified by the following scheme: 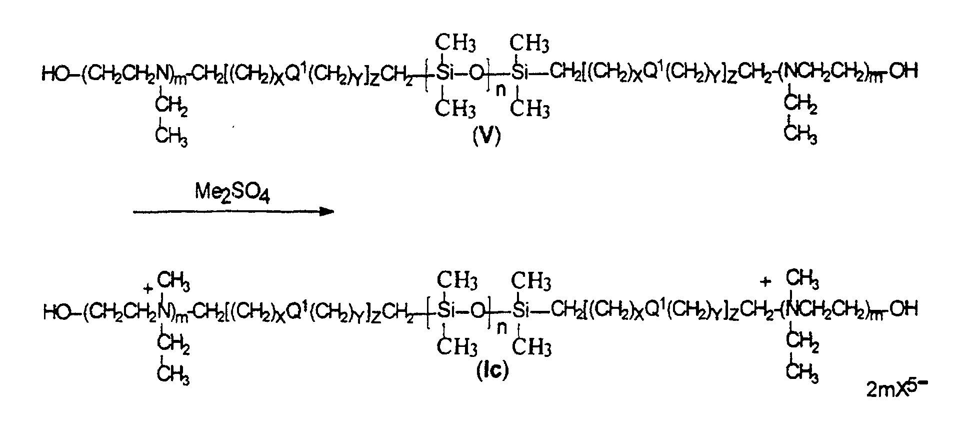
- X⁵⁻ =: mixture of anions selected from the group consisting of SO₄²⁻, HSO₄⁻, and CH₃SO₄⁻
On the other hand, when quaternization is conducted with quaternizing agents which provide protons to the amine moiety such as sulfuric acid, carboxylic acid, hydrochloric acid, hydrobromic acid, and hydroiodic acid, the quaternized cite is pH dependent. Quaternization which provides protons to the amine is exemplified by the following scheme: 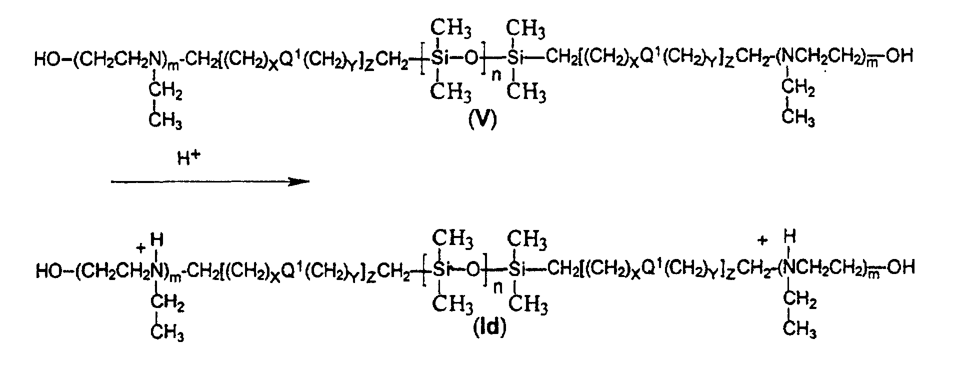 The quaternizing agent may be selected according to the desired characteristic of the final multi-cationic polymer.

### USE OF MULTI-CATIONIC POLYMER

The multi-cationic polymers of the present invention are believed to possess physical properties of both the starting polymer material and a cationic polymer. Specifically, the multi-cationic sites are believed to provide the multi-cationic polymers herein to provide good deposition and/or adhesion to various substrates such as fabrics, hair and skin, glass, metals, and filler materials. Depending on the characteristic of the starting polymer material, the multi-cationic polymer herein may further have water-miscibility, and provide antibacterial effects.

The multi-cationic polymers herein are useful in various field and employed in various products. Non-limiting examples of its useful fields include: consumer products area, medical and pharmaceutical area, fabrics area, and other industrial and chemical areas.

The multi-cationic polymers herein generally have good deposition and/or adhesion to various substrates. Thus, the multi-cationic polymer can be used as structural materials or composite materials, for coating, covering, barrier, and delivery. When formulated in a liquid system, the multi-cationic polymer may act as a flocculant, viscosity builder, or controlled-release material encapsulating actives.

### EXAMPLES

The following examples further describe and demonstrate the preferred embodiments within the scope of the present invention. The examples are given solely for the purpose of illustration and are not to be construed as limitations of the present invention since many variations thereof are possible without departing from the spirit and scope of the invention. Ingredients are identified by chemical name, or otherwise defined below.

### EXAMPLE 1

The polymer with formula (le) is suitably obtained by a synthesis route as shown in the following scheme: 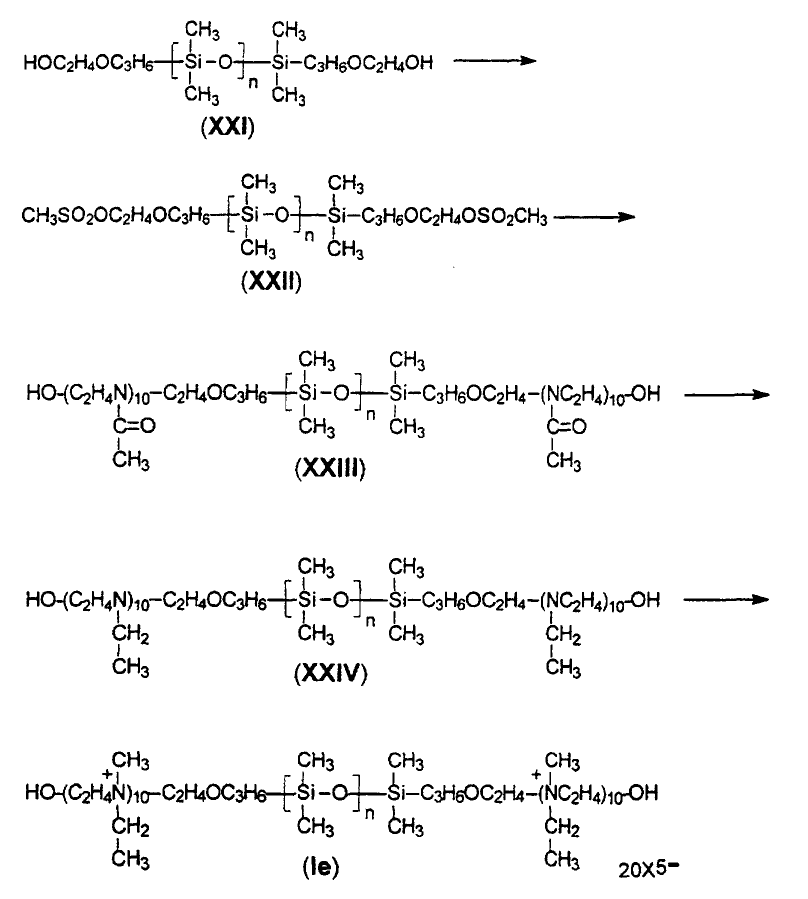
- X⁵⁻ =: mixture of anions selected from the group consisting of SO₄²⁻, HSO₄⁻, and CH₃SO₄⁻

### Activation

A 60 mmole portion of mesyl chloride (1.5 equivalent) is added dropwise to an ice-cooled stirring solution of 20 mmole dihydroxy polysiloxane having an average 90 to 110 units (XXI) and 60 mmole triethylamine (1.5 equivalent) in 120 ml of abs. THF. The reaction mixture is stirred for 1 hour under ice-cooling and for 1 day at room temperature. Removal of THF in vacuo after filtration is followed by the addition of 100 ml of ice water to the residue. This mixture is extracted with chloroform and the chloroform extract is washed with aq. sat. NaHCO₃ and dried with anhydrous sodium sulfate. This syrup is further treated according to common treatment to obtain a mesyl derivative of polysiloxane (XXII).

### Ring-opening polymerization

A sealed reaction mixture of a 5.440 g portion of the obtained mesyl siloxane (XXII) (5 mmole), 100 mmole distilled 2-methyl-2-oxazoline and 10 mmole triethylamine in 50 ml of anhydrous acetonitrile in a sealed tube is stirred at 80°C for 24 hours. The reaction mixture is then cooled to room temperature and 20 ml of 10% sodium carbonate is added with stirring. The mixture is stirred for 2 hours, then the mixture is concentrated in vacuo. The mixture is extracted with chloroform and the chloroform extract is dried with anhydrous sodium sulfate. This syrup is further treated according to common treatment to obtain a polyamide siloxane (XXIII).

### Reduction

An abs. benzene (50 ml) solution of the obtained polyamide siloxane (XXIII) (0.91 mmole) is added dropwise to an ice-cooled stirring mixture of 29 mmole (3.2 equivalent) lithium aluminum hydride and 50 ml of abs. THF. After the addition is complete the reaction mixture is stirred in ice bath for an additional hour, then the temperature is increased gradually until the reaction mixture reaches reflux, then continued stirring under reflux for 1 day. The reaction mixture is then cooled to room temperature and a 10 ml of water is added with stirring. The mixture is stirred for an hour, then the mixture is filtered. The residue is washed with hot chloroform and is extracted with chloroform under reflux 3 times. The combined filtrate are dried with anhydrous sodium sulfate. This syrup is further treated according to common treatment to obtain a N-alkyl polyethyleneimino siloxane (XXIV).

### Quaternization

A 8.1 g portion of the obtained N-alkyl polyethyleneimino siloxane (XXIV) is dissolved in 50 ml hexane and is treated with 3.2 g sodium hydrogen carbonate and 20 ml of water in ice bath cooling. To this mixture is added 4.4 g of dimethyl sulfate dropwise over a period of twenty minutes at 0°C. After the addition is complete the reaction mixture is stirred in ice bath for an additional hour, then the temperature is increased gradually until the reaction mixture reaches reflux, then continued stirring under reflux overnight. The reaction mixture is then cooled to room temperature and the hexane layer is separated and washed with sat. aq. NaHCO₃ and water 3 times. The aqueous layer is extracted with chloroform. The combined hexane extracts and chloroform extracts are dried with anhydrous sodium sulfate. This syrup is further treated according to common treatment to obtain a multi-cationic polymer (le).

### EXAMPLE 2

The polymer with formula (If) is suitably obtained using the polyamide siloxane XXIII as shown in Example 1 by a synthesis route as shown in the following scheme:

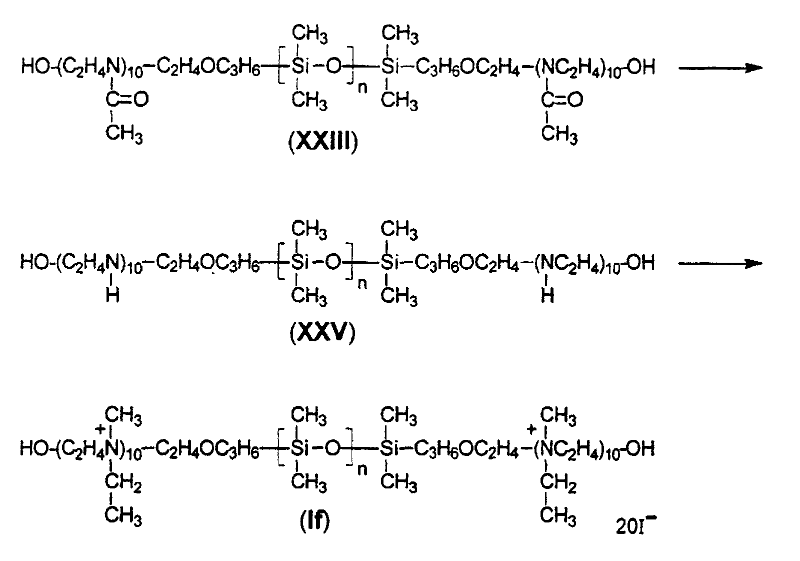

### Hydrolysis

A 20 mL portion of triethyloxonium tetrafluoroborate (1M in dichloromethane) is added dropwise over a thirty minute period to a dichloromethane solution (100 mL) of 2.0 g of polyamide siloxane (XXIII) (0.8 mmole, 1.6 mmole equivalent). The reaction mixture was allowed to stir at room temperature for 24 hours. The dichloromethane slurry of polyiminiumsiloxane salt is poured slowly over 50 mL ice-cold solution of 10% Na₂CO₃. Any solids remaining in the flask are treated with 30 mL of 10% Na₂CO₃ and combined with the original mixture. The entire mixture is extracted with dichloromethane and dried with Na₂SO₄. This syrup is further treated according to common treatment to give a two-phased oil which is identified spectroscopically as formula (XXV).

### Quaternization

A 1.5 g portion of (XXV) was dissolved in 50 mL abs. ethanol and was treated with 2 g of potassium hydrogen carbonate. To this mixture is added a mixture of 7 g methyl iodide in 10 mL abs. ethanol dropwise over a period of twenty minutes. The reaction mixture was allowed to stir at room temperature for 24 hours. Solvent of this solution was removed in vacuo to give a solid material whose spectroscopic data identified the material as multi-cationic polymer of formula (If).

### EXAMPLE 3

The polymer with formula (Ig) is suitably obtained by a synthesis route as shown in the following scheme: 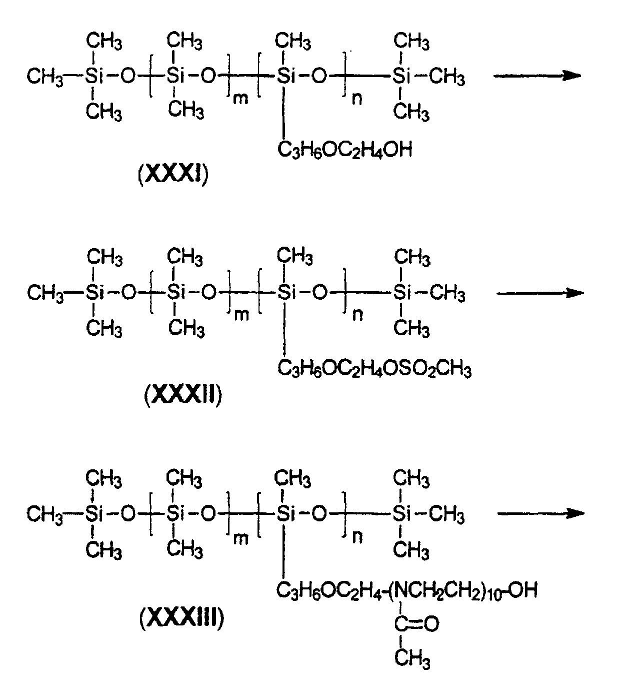 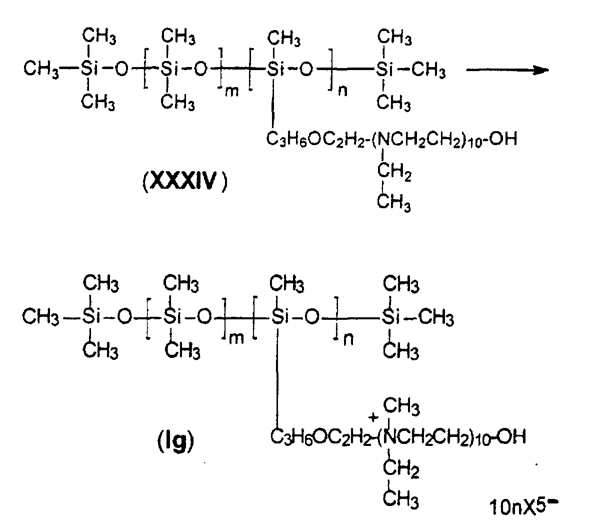
- X⁵⁻ =: mixture of anions selected from the group consisting of SO₄²⁻, HSO₄⁻, and CH₃SO₄⁻

### Activation

A 90 mmole portion of mesyl chloride (1.5 equivalent) is added dropwise to an ice-cooled stirring solution of 20 mmole branched carbinol polysiloxane having an average 50 to 70 units (structure XXXI) and 90 mmole triethylamine (1.5 equivalent) in 120 ml of abs. THF. The reaction mixture is stirred for 1 hour under ice-cooling and for 1 day at room temperature. Removal of THF in vacuo after filtration is followed by the addition of the 100 ml of ice water to the residue. This mixture is extracted with chloroform 3 times and the combined chloroform extracts are washed with aq. sat. NaHCO₃ sol. and dried with anhydrous sodium sulfate. This syrup is further treated according to common treatment to obtain a mesyl derivative of polysiloxane (XXXII).

### Ring-opening polymerization

A sealed reaction mixture of a 5 mmole portion of the obtained mesyl siloxane (XXXII), 150 mmole distilled 2-methyl-2-oxazoline and 15 mmole triethylamine in 50 ml of anhydrous acetonitrile in a sealed tube is stirred at 80°C for 24 hours. The reaction mixture is then cooled to room temperature and 20 ml of 10% sodium carbonate is added with stirring. The mixture is stirred for 2 hours, then the mixture is concentrated in vacuo. The mixture is extracted with chloroform and the chloroform extract is dried with anhydrous sodium sulfate. This syrup is further treated according to common treatment to obtain a polyamide siloxane (XXXIII).

### Reduction

An abs. benzene (50 ml) solution of the obtained polyamide siloxane (XXXIII) (0.91 mmole) is added dropwise to an ice-cooled stirring mixture of 29 mmole (3.2 equivalent) lithium aluminum hydride and 50 ml of abs. THF. After the addition is completed the reaction mixture is stirred in ice bath for an additional hour, then the temperature is increased gradually until the reaction mixture reaches reflux, then continued stirring under reflux for 1 day. The reaction mixture is then cooled to room temperature and 10 ml of water is added with stirring. The mixture is stirred for a hour, then the mixture is filtered. The residue is washed with hot chloroform and is extracted with chloroform under reflux 3 times. The combined filtrates are dried with anhydrous sodium sulfate. This syrup is further treated according to common treatment to obtain a N-alkyl polyethyleneimino siloxane (XXXIV).

### Quaternization

A 8.1 g portion of the obtained N-alkyl polyethyleneimino siloxane (XXXIV) is dissolved in 50 ml hexane and is treated with 3.2 g of sodium hydrogen carbonate and 20 ml of water in ice bath cooling. To this mixture is added 4.4 g of dimethyl sulfate dropwise over a period of twenty minutes at 0°C. After the addition is complete the reaction mixture is stirred in ice bath for an additional hour, then the temperature is increased gradually until the reaction mixture reaches reflux, then continued stirring under reflux overnight. The reaction mixture is then cooled to room temperature and the hexane layer is separated and washed with sat. aq. NaHCO₃ and water 3 times. The aqueous layer is extracted with chloroform. The combined hexane extracts and chloroform extracts are dried with anhydrous sodium sulfate. This syrup is further treated according to common treatment to obtain a multi-cationic polymer (Ig).

### EXAMPLE 4

The polymer with formula (Ih) is suitably obtained by a synthesis route as shown in the following scheme prepared by starting material (XLI) and the same reacting agents, solvents, and conditions as described in Example 3 above. 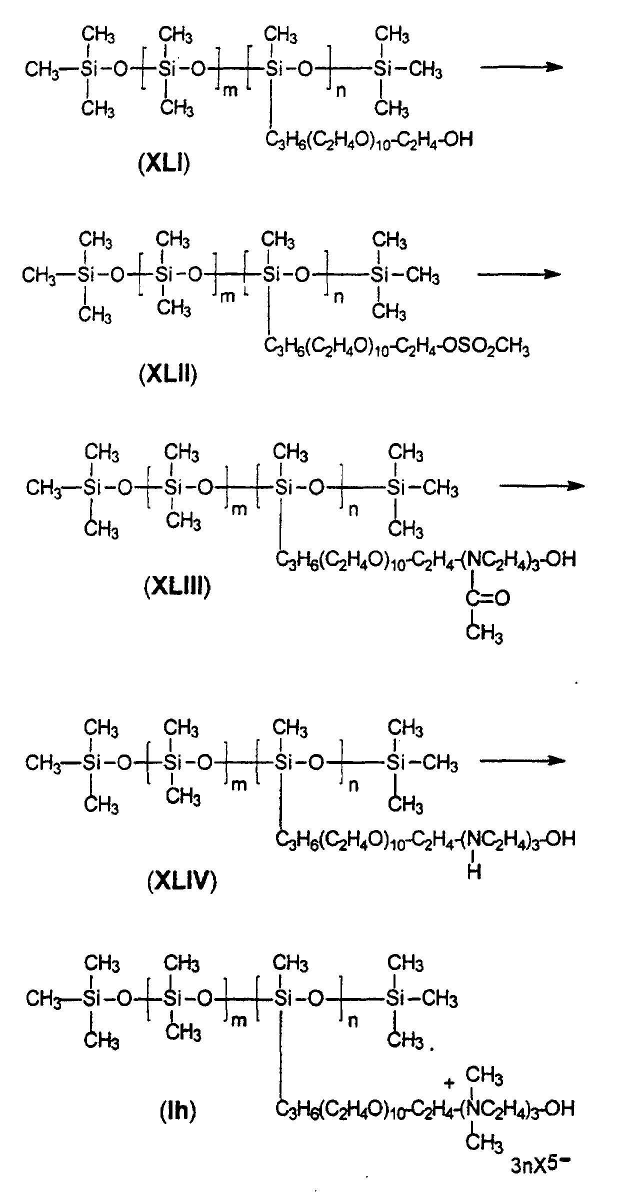
- X⁵⁻ =: mixture of anions selected from the group consisting of SO₄²⁻, HSO₄⁻, and CH₃SO₄⁻

### EXAMPLE 5

The polymer with formula (Ij) is suitably obtained by a synthesis route as shown in the following scheme: 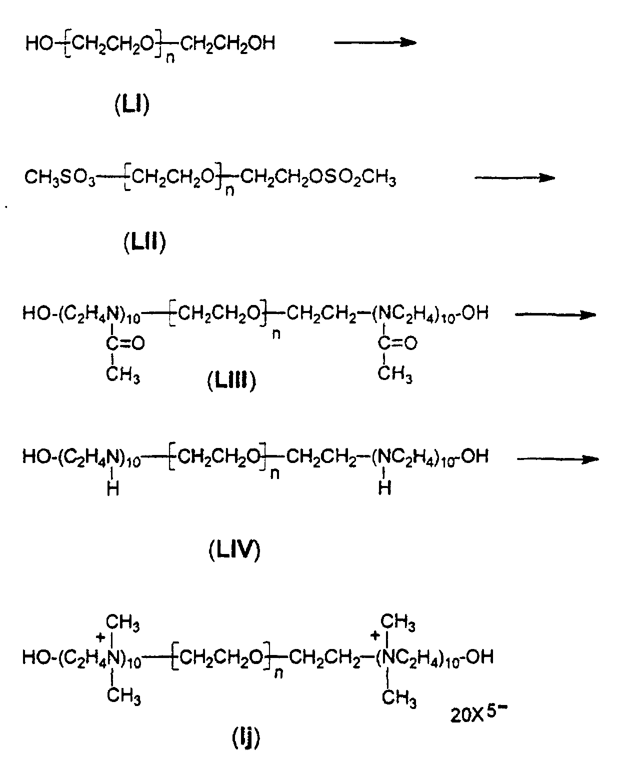
- X⁵⁻ =: mixture of anions selected from the group consisting of SO₄²⁻, HSO₄⁻, and CH₃SO₄⁻

### Activation

In a 1000mL flask, a 0.15 mole portion of mesyl chloride (1.72g) is added via addition funnel to a stirred magnetic solution of polyethylene oxide having 10 to 11 units (structure LI) in 0.15 mole triethylamine (15.2g) in 150 ml of abs. THF under cooling with ice water. After the addition, the reaction mixture is stirred for 2 hours under ice-cooling and then at room temperature for 1 day. The mixture is filtered, and the precipitate is washed by abs. THF. To the solution, 100mL of a saturated solution of NaHCO₃ is added and stirred at room temperature for 2 hours. The solution is extracted with 100mL chloroform 3 times. The chloroform phases are combined and dried over anhydrous sodium sulfate. After removal of the dry agent, chloroform solvent is removed at 35°C *in vacuo.* The residue remining is heated at 60°C in high vacuum for 2 hours to obtain a mesyl derivative of polyethylene oxide (LII).

### Ring-opening polymerization

In a 200mL tube with a magnetic stirrer, the obtained mesyl polyethylene oxide (30.3g, 0.04mol), 2-methyl-2-oxazoline (68g, 0.8mol), triethylamine (8.1g, 0.08mol) and acetonitrile (60mL) are added under nitrogen atmosphere. The tube is sealed and the mixture stirred under 80°C for 24 hours. The mixture is then cooled to room tempearture and 20 ml of 10% sodium carbonate is added with stirring. The mixture is stirred for 2 hours, and filtered. The mixture is treated under vacuum to remove the acetonitrile and water. The product is purifed by dissolving in chloroform and precipitating in isopropanol to obtain a polyamide polyethylene oxide (LIII).

### Hydrolysis

In a 100mL flask with a condenser, the obtained polyamide polyethylene oxide (LIII) (69g, 0.6mol) is dissolved in 180mL water. An aqueous solution of NaOH (36g, 0.9mol) is added under stirring. The mixture is stirred at 100°C for 24 hours. After cooling to room temperature, insoluble product is isolated by filtration, and extracted with methanol. The methanol is evaporated to opbtain N-alkyl polyethyleneimino polyethylene oxide (LIV).

### Quaternization

In a 1000mL 3-necked flask, dimethyl sulfate (151.2g, 1.2 mol) and 100mL methanol mixture is slowly dropped to a solution of the obtained N-alkyl polyethyleneimino polyethylene oxide (LIV) (43.8g, 0.6 mol) and methanol (200ml) with magnetic stirring under cooling with ice water. Then the mixture is stirred at room temperature for 24 hours. The mixture is poured into isopropanol, and the precipitated product is separated by filtration. The product is dried in vacuum at 60°C for 24 hours to obtain a multi-cationic polymer (Ij).

### EXAMPLE 6

The polymer with formula (Ik) is suitably obtained by a synthesis route as shown in the following scheme prepared by starting material (LXI) and the same reacting agents, solvents, and conditions as described in Example 5 above.

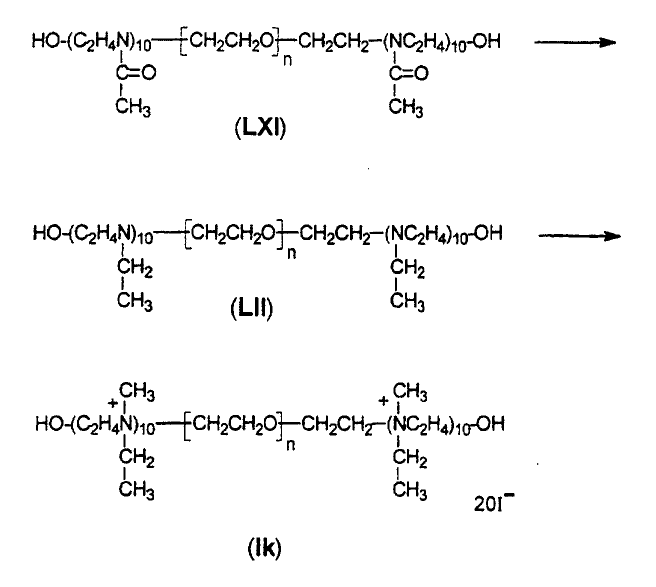

### EXAMPLES 7 - 22

The following polymers were prepared by a suitable method as selected by the artisan by the reading of the present specification.

### Example 7

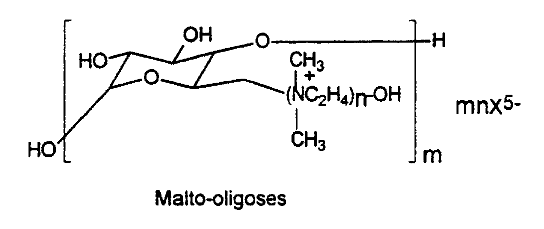

wherein m is 6, n is 5, and X⁵⁻ is a mixture of anions selected from the group consisting of SO₄²⁻, HSO₄⁻, and CH₃SO₄⁻.

### Example 8

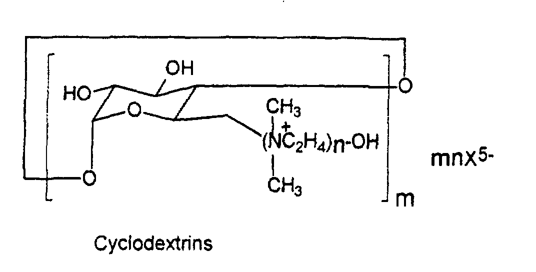

wherein m is 6, n is 3, and X⁵⁻ is a mixture of anions selected from the group consisting of SO₄²⁻, HSO₄⁻, and CH₃SO₄⁻.

### Example 9

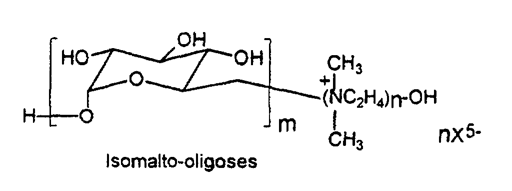

wherein m is 6, n is 5, and X⁵⁻ is a mixture of anions selected from the group consisting of SO₄²⁻, HSO₄⁻, and CH₃SO₄⁻.

### Example 10

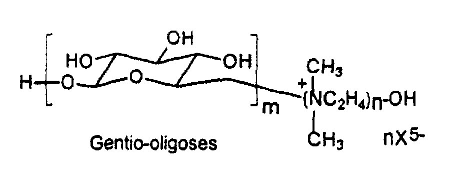

wherein m is 4, n is 5, and X⁵⁻ is a mixture of anions selected from the group consisting of SO₄²⁻, HSO₄⁻, and CH₃SO₄⁻.

### Example 11

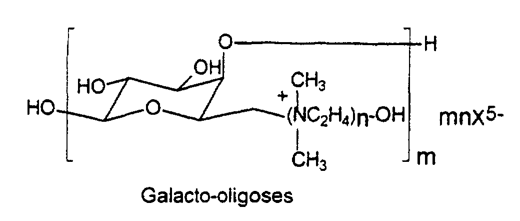

wherein m is 4, n is 5, and X⁵⁻ is a mixture of anions selected from the group consisting of SO₄²⁻, HSO₄⁻, and CH₃SO₄⁻.

### Example 12

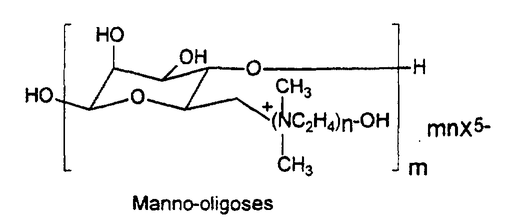

wherein m is 4, n is 2, and X⁵⁻ is a mixture of anions selected from the group consisting of SO₄²⁻, HSO₄⁻, and CH₃SO₄⁻.

### Example 13

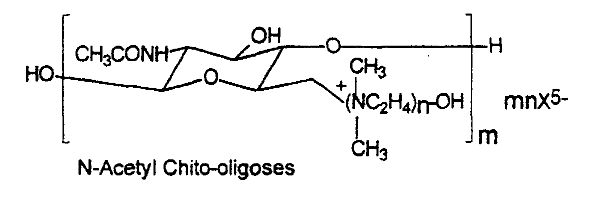

wherein m is 5, n is 3, and X⁵⁻ is a mixture of anions selected from the group consisting of SO₄²⁻, HSO₄⁻, and CH₃SO₄⁻.

### Example 14

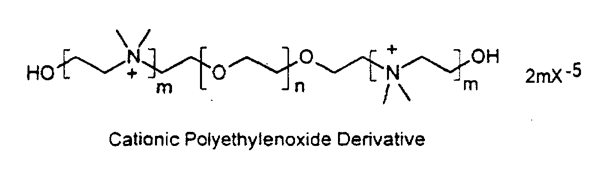

wherein m is 10, n is 10 to 13, and X⁵⁻ is a mixture of anions selected from the group consisting of SO₄²⁻, HSO₄⁻, and CH₃SO₄⁻

### Example 15

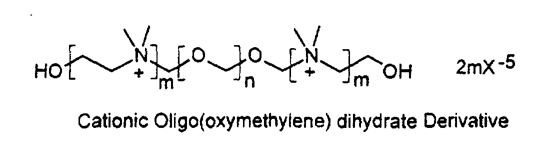

wherein m is 10, n is 10 to 15, and X⁵⁻ is a mixture of anions selected from the group consisting of SO₄²⁻, HSO₄⁻, and CH₃SO₄⁻

### Example 16

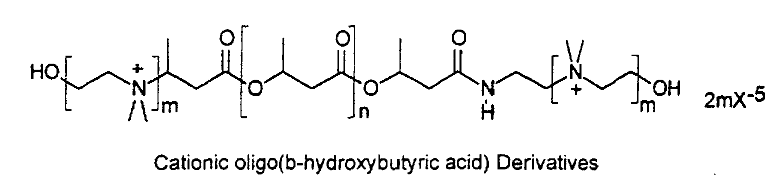

wherein m is 10, n is 30 to 50, and X⁵⁻ is a mixture of anions selected from the group consisting of SO₄²⁻, HSO₄⁻, and CH₃SO₄⁻

### Example 17

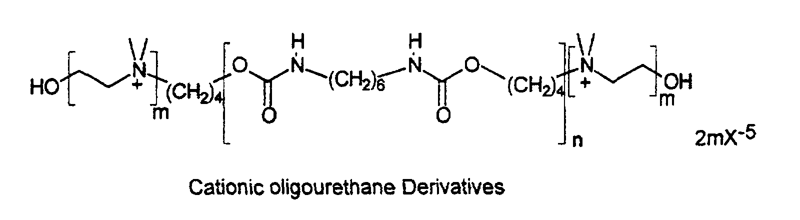

wherein m is 10, n is 30 to 50, and X⁵⁻ is a mixture of anions selected from the group consisting of SO₄²⁻, HSO₄⁻, and CH₃SO₄⁻

### Example 18

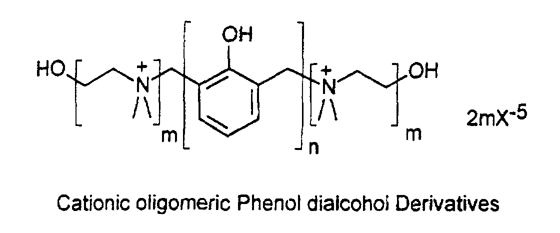

wherein m is 10, n is 10 to 15, and X⁵⁻ is a mixture of anions selected from the group consisting of SO₄²⁻, HSO₄⁻, and CH₃SO₄⁻

### Example 19

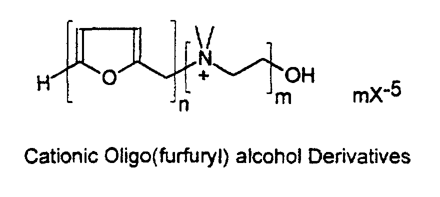

wherein m is 10, n is 30 to 50, and X⁵⁻ is a mixture of anions selected from the group consisting of SO₄²⁻, HSO₄⁻, and CH₃SO₄⁻

### Example 20

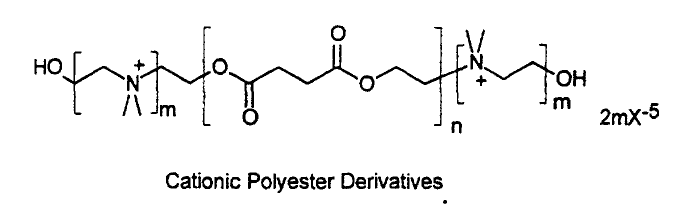

wherein m is 10, n is 30 to 50, and X⁵⁻ is a mixture of anions selected from the group consisting of SO₄²⁻, HSO₄⁻, and CH₃SO₄⁻

### Example 21

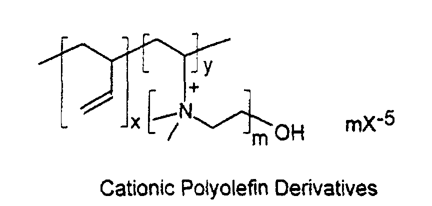

wherein m is 10, x is 30 to 50, y is 30 to 50, and X⁵⁻ is a mixture of anions selected from the group consisting of SO₄²⁻, HSO₄⁻, and CH₃SO₄⁻.

### Example 22

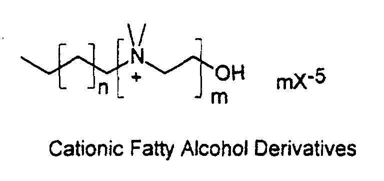

wherein m is 5, n is 10 to 30, and X⁵⁻ is a mixture of anions selected from the group consisting of SO₄²⁻, HSO₄⁻, and CH₃SO₄⁻.

The multi-cationic polymer embodiments disclosed and represented by the previous examples have many advantages.

Multi-cationic polymers obtained by Examples 1 through 4 provide, for example, good deposition to the surface of fabric and hair, and provide bacteriocide effects when included in aqueous compositions. When incorporated in fabric treatment compositions, they provide anti-wrinkle benefit, softness, and anti-static benefit. When incorporated in hair conditioner compositions, they provide conditioning benefit and anti-static benefit.

## Claims

1. A multi-cationic polymer comprising:
a) formulae (Ia): 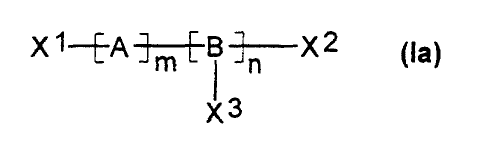 wherein [A]ₘ and [B]ₙ, individually, is a non-reacting polymeric substituent wherein m+n is an integer of 1 to about 100,000, where one of m or n can be 0; and at least one of X¹, X², and X³ is formulae (Ib): 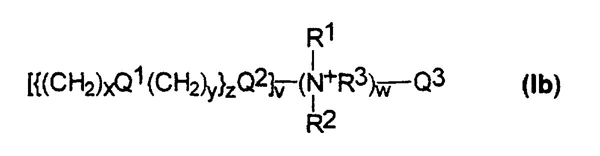 wherein Q¹ is oxygen, nitrogen, sulfur or methylene; x is an integer of 0 to about 20; y is an integer of 0 to about 20; z is an integer of 0 to about 20; Q² is CH₂ or any of the following formulae: 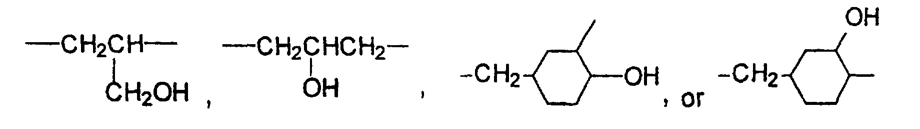 v is 0 or 1; R¹ is hydrogen, alkyl of 1 to 4 carbons, fluorocarbon of 1 to 4 carbons, phenyl, or benzyl; R² is alkyl of 1 to about 30 carbons, phenyl, benzyl, or phenethyl; R³ is ethylene or propylene; Q³ is OH, OR⁸, NH₂, NHR⁹, SH, SR¹⁰, COOH, COOR¹¹, or a halogen, wherein R⁸, R⁹, R¹⁰, and R¹¹ are alkyl or alkylene of 1 to about 20 carbons; and w is an integer of 1 to about 100; the remainder of X¹, X², and X³ being a non-reacting end group; and
b) a stoichiometric amount of an anion selected from the group consisting of anionic forms of sulfate, hydrosulfate, methylsulfate, carbonate, bicarbonate, chloride, bromide, iodide, and mixtures thereof.

2. The multi-cationic polymer according to Claim 1 wherein [A]ₘ and [B]ₙ, individually, are polysiloxanes, polyalkylene oxides, polysaccharides, oligo(oxymethylene) dihydrates, oligo(esters) of hydroxy acids, diol oligo(urethanes), polymeric phenol alcohols, poly(furfuryl) derivatives, polyacrylates, polyesters, polyolefins, poly(vinyl alcohol)s, long alkyl fatty alcohols, polyols, and mixtures thereof.

3. The multi-cationic polymer according to Claim 2 wherein R¹ is hydrogen, methyl, ethyl, phenyl, or benzyl; R² is alkyl of 1 to about 30 carbons; R³ is ethylene or propylene; w is 3 to about 100; and Q³ is OH.

4. The multi-cationic polymer according to Claim 1 wherein [A]ₘ and [B]ₙ, individually, are polyalkylene oxides selected from the group consisting of poly(methylene oxide), poly(ethylene oxide), poly(propylene oxide), poly(butylene oxide), monoalkylethers of poly(alkylene oxides), and mixtures thereof; v is 0; R¹ is hydrogen, methyl, ethyl, phenyl, or benzyl; R² is alkyl of 1 to about 30 carbons; R³ is ethylene or propylene; w is 3 to about 100; and Q³ is OH.

5. The multi-cationic polymer according to Claim 1 wherein [A]ₘ and [B]ₙ, individually, are polysaccharides selected from the group consisting of poly(β-xylopyranose), poly(maltose), cyclodextrins, celluloses, poly(isomaltose), poly(gentiobiose), poly(galactose), poly(mannose), chitin and mixtures thereof; v is 0; R¹ is hydrogen, methyl, ethyl, phenyl, or benzyl; R² is alkyl of 1 to about 30 carbons; R³ is ethylene or propylene; w is 3 to about 100; and Q³ is OH.

6. A method of making the multi-cationic polymer of Claim 1 comprising the steps of:
(a) ring-opening polymerization of an activated starting polymer with a cyclic iminoether selected from the group consisting of 2-substituted-2-oxazoline, 2-substituted-5,6-dihydro-4H 1,3-oxazine, and mixtures thereof;
(b) reduction or hydrolysis of the compound formed in step (a); and
(c) quaternization of the compound formed in step (b).

7. The method according to Claim 6 wherein step (a) comprises a preliminary step of making the activated starting polymer, the preliminary step comprising reacting a starting material with an activating agent selected from the group consisting of tosyl halide, mesyl halide, and mixtures thereof; wherein the starting material has at least one hydroxy functionality terminal, the remainder terminals being non-reactive with the activating agents.

8. The method according to Claim 6 wherein the cyclic iminoether is selected from the group consisting of 2-Z'-2-oxazoline, 2-Z'-oxazine, and mixtures thereof; wherein Z' is a hydrogen, alkyl having 1 to 10 carbons, fluorocarbon having 1 to 10 carbons, phenyl, or benzyl.

9. The method according to Claim 8 wherein the cyclic iminoether is selected from the group consisting of 2-methyl-2-oxazoline, 2-ethyl-2-oxazoline, 2-propyl-2-oxazoline, and mixtures thereof.

10. The method according to Claim 6 wherein at least a stoichiometric amount to the activated starting polymer of a Lewis base is used as a solvent medium at step (a).

## Patentansprüche

1. Multikationisches Polymer, umfassend:
a) Formeln (Ia): 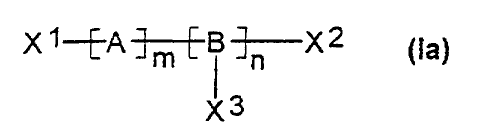 worin [A]ₘ und [B]ₙ jeweils einzeln ein nichtreagierender, polymerer Substituent ist, worin m+n eine ganze Zahl von 1 bis etwa 100.000 ist, wobei eines von m oder n 0 sein kann; und mindestens eines von X¹, X² und X³ der Formel (Ib) entspricht: 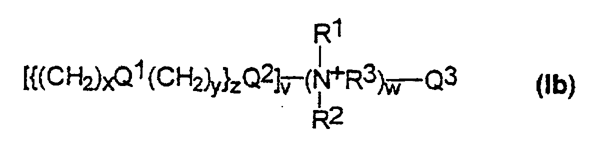 worin Q¹ Sauerstoff, Stickstoff, Schwefel oder Methylen ist; x eine ganze Zahl von 0 bis etwa 20 ist; y eine ganze Zahl von 0 bis etwa 20 ist; z eine ganze Zahl von 0 bis etwa 20 ist; Q² CH₂ oder irgend eine der folgenden Formeln ist; 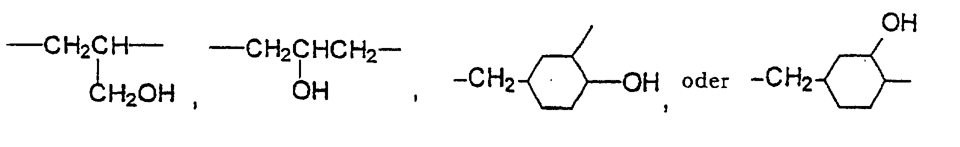 v 0 oder 1 ist; R¹ Wasserstoff, Alkyl mit 1 bis 4 Kohlenstoffatomen, Fluorkohlenstoff mit 1 bis 4 Kohlenstoffatomen, Phenyl oder Benzyl ist; R² Alkyl mit 1 bis etwa 30 Kohlenstoffatomen, Phenyl, Benzyl oder Phenethyl ist; R³ Ethylen oder Propylen ist; Q³ OH, OR⁸, NH₂, NHR⁹, SH, SR¹⁰, COOH, COOR¹¹ oder ein Halogen ist, worin R⁸, R⁹, R¹⁰ und R¹¹ Alkyl oder Alkylen mit 1 bis etwa 20 Kohlenstoffatomen sind; und w eine ganze Zahl von 1 bis etwa 100 ist; wobei der Rest von X¹, X² und X³ eine nichtreagierende Endgruppe sind; und
b) eine stöchiometrische Menge eines Anions, gewählt aus der anionische Formen von Sulfat, Hydrosulfat, Methylsulfat, Carbonat, Bicarbonat, Chlorid, Bromid, Iodid und Mischungen hiervon umfassenden Gruppe.

2. Multikationisches Polymer nach Anspruch 1, worin [A]m und [B]n einzeln Polysiloxane, Polyalkylenoxide, Polysaccharide, Oligo(oxymethylen)dihydrate, Oligo(ester) von Hydroxysäuren, Diololigo(urethane), polymere Phenolalkohole, Poly(furfuryl)-Derivate, Polyacrylate, Polyester, Polyolefine, Poly(vinylalkohol)e, lange Alkylfettalkohole, Polyole und Mischungen hiervon sind.

3. Multikationisches Polymer nach Anspruch 2, wobei R¹ Wasserstoff, Methyl, Ethyl, Phenyl oder Benzyl ist; R² Alkyl mit 1 bis etwa 30 Kohlenstoffatomen ist; R³ Ethylen oder Propylen ist; w 3 bis etwa 100 ist; und Q³ OH ist.

4. Multikationisches Polymer nach Anspruch 1, worin [A]ₘ und [B]ₙ einzeln Polyalkylenoxide sind, gewählt aus der Gruppe, bestehend aus Poly(methylenoxid), Poly(ethylenoxid), Poly(propylenoxid), Poly(butylenoxid), Monoalkylether von Poly(alkylenoxiden) und Mischungen hiervon; v 0 ist; R¹ Wasserstoff, Methyl, Ethyl, Phenyl oder Benzyl ist; R² Alkyl mit 1 bis etwa 30 Kohlenstoffatomen ist; R³ Ethylen oder Propylen ist; w 3 bis etwa 100 ist; und Q³ OH ist.

5. Multikationisches Polymer nach Anspruch 1, wobei [A]ₘ und [B]ₙ einzeln Polysaccharide sind, gewählt aus der Gruppe, bestehend aus Poly(β-xylopyranose), Poly(maltose), Cyclodextrinen, Cellulosen, Poly(isomaltose), Poly(gentiobiose), Poly(galactose), Poly(mannose), Chitin und Mischungen hiervon; v O ist; R¹ Wasserstoff, Methyl, Ethyl, Phenyl oder Benzyl ist; R² Alkyl mit 1 bis etwa 30 Kohlenstoffatomen ist; R³ Ethylen oder Propylen ist; w 3 bis etwa 100 ist; und Q³ OH ist.

6. Verfahren zur Herstellung des multikationischen Polymeren nach Anspruch 1, umfassend die Schritte:
(a) Ringöffnungspolymerisation eines aktivierten Ausgangspolymeren mit einem cyclischen Iminoether, gewählt aus der Gruppe, bestehend aus 2-substituiertem-2-Oxazolin, 2-substituiertem-5,6-Dihydro-4-H 1,3-oxazin und Mischungen hiervon;
(b) Reduktion oder Hydrolyse der in Schritt (a) gebildeten Verbindung; und
(c) Quaternisierung der in Schritt (b) gebildeten Verbindung.

7. Verfahren nach Anspruch 6, wobei Schritt (a) einen vorausgehenden Schritt des Herstellens des aktivierten Ausgangspolymeren umfaßt, wobei der vorausgehende Schritt das Umsetzen eines Ausgangsmaterials mit einem Aktivierungsmittel umfaßt, gewählt aus der Gruppe, bestehend aus Tosylhalogenid, Mesylhalogenid, und Mischungen hiervon; wobei das Ausgangsmaterial mindestens ein hydroxyfunktionelles Ende aufweist, wobei die verbleibenden Enden mit den Aktivierungsmitteln nicht reaktiv sind.

8. Verfahren nach Anspruch 6, wobei der cyclische Iminoether aus der Gruppe gewählt ist, bestehend aus 2-Z'-2-Oxazolin, 2-Z'-Oxazin und Mischungen hiervon; worin Z' Wasserstoff, Alkyl mit 1 bis 10 Kohlenstoffatomen, Fluorkohlenstoff mit 1 bis 10 Kohlenstoffatomen, Phenyl oder Benzyl ist.

9. Verfahren nach Anspruch 8, wobei der zyklische Iminoether gewählt ist aus der Gruppe, bestehend aus 2-Methyl-2-oxazolin, 2-Ethyl-2-oxazolin, 2-Propyl-2-oxazolin und Mischungen hiervon.

10. Verfahren nach Anspruch 6, wobei mindestens eine stöchiometrische Menge des aktivierten Ausgangspolymeren einer Lewisbase als Lösungsmittelmedium beim Schritt (a) verwendet wird.

## Revendications

1. Polymère polycationique comprenant :
a) la formule (Ia) : 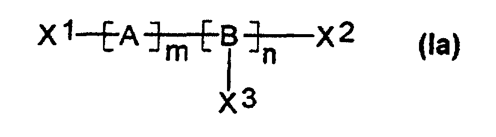 dans laquelle [A]ₘ et [B]ₙ, individuellement, représentent un substituant polymère non réactif dans lequel m+n est un entier valant 1 à environ 100 000, où un des m ou n peut valoir 0 ; et au moins un des X¹, X², et X³ répond à la formule (Ib) : 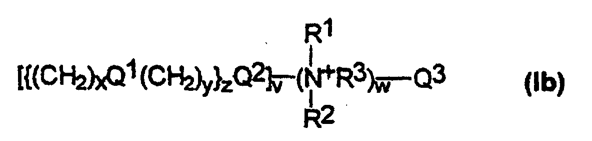 dans laquelle Q¹ est un oxygène, un azote, un soufre ou un méthylène ; x est un entier valant 0 à environ 20 ; y est un entier valant 0 à environ 20 ; z est un entier valant 0 à environ 20 ; Q² est CH₂ ou répond à l'une quelconque des formules suivantes : 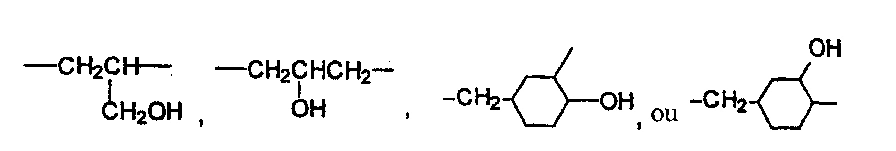 v vaut 0 ou 1 ; R¹ est un hydrogène, un alkyle de 1 à 4 atomes de carbone, un fluorocarbure de 1 à 4 atomes de carbone, un phényle, ou un benzyle ; R² est un alkyle de 1 à environ 30 atomes de carbone, un phényle, un benzyle, ou un phénéthyle ; R³ est un éthylène ou un propylène ; Q³ est OH, OR⁸, NH₂, NHR⁹, SH, SR¹⁰, COOH, COOR¹¹, ou un halogène, où R⁸, R⁹, R¹⁰, et R¹¹ sont des groupes alkyle ou alkylène de 1 à environ 20 atomes de carbone ; et w est un entier valant 1 à environ 100 ; le reste des X¹, X², et X³ étant un groupe terminal non réactif ; et
b) une quantité stoechiométrique d'un anion choisi dans le groupe constitué des formes anioniques du sulfate, hydrosulfate, méthylsulfate, carbonate, bicarbonate, chlorure, bromure, iodure, et leurs mélanges.

2. Polymère polycationique selon la revendication 1 dans lequel [A]ₘ et [B]ₙ, individuellement, sont des polysiloxanes, des oxydes de polyalkylène, des polysaccharides, des dihydrates d'oligo(oxyméthylène), des oligo(esters) d'hydroxyacides, des diol-oligo(uréthanes), des alcools phénoliques polymères, des dérivés de poly(furfuryle), des polyacrylates, des polyesters, des polyoléfines, des poly(alcools vinyliques), des alcools gras à longue chaîne alkyle, des polyols, et leurs mélanges.

3. Polymère polycationique selon la revendication 2 dans lequel R¹ est un hydrogène, un méthyle, un éthyle, un phényle, ou un benzyle ; R² est un alkyle de 1 à environ 30 atomes de carbone ; R³ est un éthylène ou un propylène ; w vaut 3 à environ 100 ; et Q³ est OH.

4. Polymère polycationique selon la revendication 1 dans lequel [A]ₘ et [B]ₙ, individuellement, sont des oxydes de polyalkylène choisis dans le groupe constitué du poly(oxyde de méthylène), du poly(oxyde d'éthylène), du poly(oxyde de propylène), du poly(oxyde de butylène), des monoalkyléthers de poly(oxydes d'alkylène), et de leurs mélanges ; v vaut 0 ; R¹ est un hydrogène, un méthyle, un éthyle, un phényle, ou un benzyle ; R² est un alkyle de 1 à environ 30 atomes de carbone ; R³ est un éthylène ou un propylène ; w vaut 3 à environ 100 ; et Q³ est OH.

5. Polymère polycationique selon la revendication 1 dans lequel [A]ₘ et [B]ₙ, individuellement, sont des polysaccharides choisis dans le groupe constitué du poly(β-xylopyranose), du poly(maltose), des cyclodextrines, des celluloses, du poly(isomaltose), du poly(gentiobiose), du poly(galactose), du poly(mannose), de la chitine et de leurs mélanges ; v vaut 0 ; R¹ est un hydrogène, un méthyle, un éthyle, un phényle, ou un benzyle ; R² est un alkyle de 1 à environ 30 atomes de carbone ; R³ est un éthylène ou un propylène ; w vaut 3 à environ 100 ; et Q³ est OH.

6. Procédé de préparation du polymère polycationique de la revendication 1 comprenant les étapes de :
(a) polymérisation par ouverture de cycle d'un polymère de départ activé, avec un iminoéther cyclique choisi dans le groupe constitué de la 2-oxazoline ayant un substituant en position 2, de la 5,6-dihydro-4H-1,3-oxazine ayant un substituant en position 2, et de leurs mélanges ;
(b) réduction ou hydrolyse du composé formé dans l'étape (a) ; et
(c) quaternisation du composé formé dans l'étape (b).

7. Procédé selon la revendication 6 dans lequel l'étape (a) comprend une étape préliminaire de préparation du polymère de départ activé, l'étape préliminaire comprenant la réaction d'une matière de départ avec un agent d'activation choisi dans le groupe constitué d'un halogénure de tosyle, d'un halogénure de mésyle, et de leurs mélanges ; dans lequel la matière de départ a au moins une extrémité à fonçtion hydroxy, les extrémités restantes étant non réactives avec les agents d'activation.

8. Procédé selon la revendication 6 dans lequel l'iminoéther cyclique est choisi dans le groupe constitué de la 2-Z'-2-oxazoline, de la 2-Z'-oxazine, et de leurs mélanges ; dans lequel Z' est un hydrogène, un alkyle ayant 1 à 10 atomes de carbone, un fluorocarbure ayant 1 à 10 atomes de carbone, un phényle, ou un benzyle.

9. Procédé selon la revendication 8 dans lequel l'iminoéther cyclique est choisi dans le groupe constitué de la 2-méthyl-2-oxazoline, de la 2-éthyl-2-oxazoline, de la 2-propyl-2-oxazoline, et de leurs mélanges.

10. Procédé selon la revendication 6 dans lequel au moins une quantité stoechiométrique par rapport au polymère de départ activé d'une base de Lewis est utilisée comme milieu solvant à l'étape (a).
